# EUROPEAN PATENT APPLICATION

(11) **EP 4 091 600 A1**
(43) Date of publication of application: **23.11.2022**
(21) Application number: 20914264.5
(22) Date of filing: 24.04.2020
(51) Int. Cl.: A61K 8/02, A61K 8/46, A61K 8/42, A61K 8/73, A61K 8/89, A61K 8/81, A61K 8/88, A61K 8/19

(54) **SOLID PERSONAL CARE PRODUCT AND PRODUCTION METHOD THEREOF**

(30) Priority: 17.01.2020 KR 20200006863
(71) Applicant: Blisspack Co., Ltd., Siheung-si, Gyeonggi-do 15104 (KR)
(72) Inventor: AHN, Jong Won, Seoul 06906 (KR); AN, Jae Gwan, Ansan-si Gyeonggi-do 15535 (KR); KIM, Yong Joon, Seoul 06092 (KR)
(74) Representative: Ruzzu, Giammario
(86) International application number: PCT/KR2020/005469
(87) International publication number: WO 2021/145512

(57) **Abstract**

The present invention provides a solid personal care product comprising: a core layer containing a cleansing or conditioning component; and a skin layer containing a water-soluble or water-dispersible polymer. A solid personal care product produced according to the present invention solves the disadvantage that existing conventional solid cleansing agents or conditioning agents in powder, soap, or other flake form have of the powder scattering, as well as the problem of the cleansing power, conditioning efficacy, and fragrance-preserving property of such agents degrading. Moreover, the contents of the cleansing or conditioning component does not rub off onto the hands of a user even when the user holds the contents in the user's hands, and the shape of the product according to the present invention does not change even in places of high humidity. In addition, this solid personal care product is easy to use, store, and package, while retaining the same functions as those of conventional liquid cleansing and conditioning personal care products.

## Description

### TECHNICAL FIELD

The present invention relates to a solid personal care product and a production method thereof, and more particularly, to a technique that produce a solid personal care product comprising: a core layer containing a cleansing or conditioning component; and a skin layer containing a water-soluble or water-dispersible polymer, and applies it as a cleaning agent or a conditioning agent such as facial cleanser, shampoo, conditioner, body cleanser, facial foam, and shaving foam.

### BACKGROUND ART

Generally, skin or hair is polluted by sweat and various sebum secreted through human metabolism. It is also further polluted due to various sources of atmospheric pollution and other external environmental factors. Conventional cleaning products such as shampoo or body cleanser go through a series of processes which comprises soaking the skin or hair sufficiently in water, then coating the liquid body cleanser or shampoo onto the skin or hair, massaging with foam, rinsing, drying, and trimming. Such personal care products such as shampoos and body cleansers are usually produced by dissolving or dispersing a liquid cleaning component, a gelling agent, a fragrance, a moisturizing agent, a conditioning agent, a dye and, if necessary, a preservative in water. More than 85% of the components of the personal care products prepared in this way is composed of water, which is accompanied with many distribution costs such as transportation and storage until it reaches the consumer after production. Not only such costs are included in the purchase cost of consumers and thus bring a lot of burden to consumers, but also carbon dioxide generated in the process of transportation or storage is located as a major cause of global warming. In order to solve these problems, many research and development have been conducted on solidified cleaning and conditioning products, but the developed products are in the form of soap, powder and film, which have a disadvantage that the feeling of use and efficacy are inferior to those of the existing liquid type products. In addition to the restrictions on packaging, when actually used, a lot of time and effort are required due to clumping phenomenon, or there are few bubbles and it does not show sufficient conditioning effect. In particular, the fragrance-retaining property, which is important for consumers, is significantly inferior, and commercialization is insignificant.

Therefore, the present inventors have found that 80% or more of the dried bulk including a cleaning component, a conditioning component, an essential oil, a fragrance, a dye, an inorganic/organic filler and other additives that are contained in the liquid cleaning or conditioning forms a core layer, the bulk components of the core layer do not scatter or adhere even when touched with wet hands, while helping to maintain the shape of the core layer, and it can be prepared into the formulation in a form surrounded by a skin layer which makes the appearance beautiful, whereby not only it is easy to package and carry, but it also has a sufficient scent, so when used, it can be applied as a solid personal care product with a scent level equal to or higher than that of a liquid shampoo or body cleanser. The present invention was completed on the basis of these findings.

### Prior Art Document

### Patent Document

Patent Document 1 Korean Unexamined Patent Application Publication No. 10-2016-0120470
Patent Document 2 Korean Unexamined Patent Application Publication No. 10-2017-0036397

### DETAILED DESCRIPTION OF THE INVENTION

### Technical Problem

The above disclosure has been designed to solve that above-mentioned problem, and an object of the present invention is to provide a solid personal care product which solves the disadvantage that existing conventional solid cleansing agents or conditioning agents in powder, soap, or other flake form have of the powder scattering, as well as the problem of the cleansing power, conditioning efficacy, and fragrance-preserving property of such agents degrading, and at the same time, is easy to use, store and package while retaining the same functions as those of conventional liquid cleaning and conditioning personal care products.

### Technical Solution

In order to achieve the above-mentioned object, one aspect of the present invention provides a solid personal care product comprising: a core layer containing a cleansing or conditioning component; and a skin layer containing a water-soluble or water-dispersible polymer.

The cleansing component is at least one selected from the group consisting of sodium alkyl sulfate having 12 to 14 carbon atoms, alkyl monoethanol amide having 12 to 14 carbon atoms, N-acyl glutamate, amphoteric surfactant, cationic water-soluble polymer, polyoxyalkylene fatty acid monoisopropanol amide, sodium sulfate, sodium chloride and a mixture thereof.

The cleansing component further includes an organic powder selected from polysaccharides, dimethyl polysiloxane, polymethyl methacrylate and nylon, or an inorganic powder selected from silica, kaolin, zinc oxide, titanium oxide, zeolite and calcium carbonate, or a mixture thereof.

The cleansing component further includes at least one thickener selected from the group consisting of polyacrylic acid or a derivative thereof, polyvinyl alcohol, methyl cellulose, hydroxymethyl cellulose, xanthan gum, alginic acid, carrageenan and a mixture thereof.

The conditioning component is at least one selected from the group consisting of dimethicone, cyclomethicone, aminated silicone, isoparaffin, isopropyl palmitate, isostearyl isostearate and a mixture thereof.

The water-soluble or water-dispersible polymer is at least one selected from the group consisting of polyvinyl alcohol, alginic acid, polyacrylic acid, polyvinylacrylamide, polyvinylpyrrolidone, a cellulose derivative and a mixture thereof.

The skin layer further includes a natural fibrous polymer selected from chitin, chitosan, cellulose and silk, or a synthetic fibrous polymer selected from acrylic, nylon, rayon and polyester, or a mixture thereof.

The skin layer is a coating layer formed on a surface of the core layer.

The skin layer is configured such that a water-soluble paper or a water-soluble polymer film coated with a water-soluble polymer, or a water-soluble paper laminated with a water-soluble polymer film is laminated on both surfaces of the core layer.

The water-soluble polymer film is at least one selected from the group consisting of polyvinyl alcohol, alginic acid, polyacrylic acid or a derivative thereof and polyacrylamide.

Another aspect of the present invention provides a method for producing a solid personal care product, comprising the steps of: (I) freeze-drying a liquid bulk component containing a cleaning or conditioning component to form a core layer; and (II) coating a solution containing a water-soluble or water-dispersible polymer onto the core layer, followed by freeze-drying to form a coating layer.

Another aspect of the present invention provides a method for producing a solid personal care product, comprising the steps of: (I) coating a liquid bulk component containing a cleaning or conditioning component onto a water-soluble paper coated with a water-soluble polymer, and then laminating the water-soluble paper coated with the water-soluble polymer thereon; and (II) drying the result with a hot air dryer to form a skin layer in which a water-soluble paper coated with a water-soluble polymer is laminated on both surfaces of a core layer containing a cleaning or conditioning component.

Another aspect of the present invention provides a method for producing a solid personal care product, comprising the steps of: (I) coating a bulk slurry containing a cleaning or conditioning component in a state of -5°C to -10°C onto a water-soluble paper or a water-soluble polymer film coated with a water-soluble polymer in a chamber maintained at -30°C to -50°C, and then laminating a water-soluble paper or a water-soluble polymer film coated with a water-soluble polymer thereon; and (II) drying the result with a freeze dryer to form a skin layer in which a water-soluble paper or a water-soluble polymer film coated with a water-soluble polymer is laminated on both surfaces of a core layer containing a cleaning or conditioning component.

Yet another aspect of the present invention provides a method for producing a solid personal care product, comprising the steps of: (I) coating a bulk slurry containing a cleaning or conditioning component in a state of -5°C to -10°C onto a composite film in which a water-soluble polymer film is laminated on a water-soluble paper in a chamber maintained at -30°C to -50°C; and (II) tightly adhering the film surface of the composite film so as to go to the inside and then freeze-drying to form a skin layer in which the composite film is laminated on both surfaces of a core layer containing a cleaning or conditioning component.

The ends of the four edges are sealed using heat, ultrasonic waves, high frequency or induction heating with the bulk of the skin layer film contained therein, then cut and sealed.

The solid personal care product is spherical, disc-shaped, polygonal, hexahedral, sandwich-shaped or plate-shaped.

### ADVANTAGEOUS EFFECTS

A solid personal care product produced according to the present invention solves the disadvantage that existing conventional solid cleansing agents or conditioning agents in powder, soap, or other flake form have of the powder scattering, as well as the problem of the cleansing power, conditioning efficacy, and fragrance-preserving property of such agents degrading, and at the same time, the contents of the cleansing or conditioning component does not rub off onto the hands of a user even when the user holds the contents in the user's hands, and the shape of the product does not change even in places of high humidity. In addition, this solid personal care product is easy to use, store, and package, while retaining the same functions as those of conventional liquid cleansing and conditioning personal care products.

### BRIEF DESCRIPTION OF THE DRAINGS

FIG. 1 is a schematic diagram of a solid personal care product produced in Examples 1 or 2 of the present invention;
FIG. 2 is a schematic diagram of a solid personal care product produced in Examples 3 or 4 of the present invention; and
FIG. 3 is a schematic diagram of a solid personal care product produced in Examples 5 or 6 of the present invention.

### DETAILED DESCRIPTION OF THE EMBODIMENTS

Hereinafter, the solid personal care product according to the present invention and a method for producing the same will be described in detail together with the accompanying drawings.

According to the present invention, there is provided a solid personal care product comprising: a core layer containing a cleansing or conditioning component; and a skin layer containing a water-soluble or water-dispersible polymer.

The cleansing component contained in the core layer may be at least one selected from the group consisting of sodium alkyl sulfate having 12 to 14 carbon atoms, alkyl monoethanol amide having 12 to 14 carbon atoms, N-acyl glutamate, amphoteric surfactant, cationic water-soluble polymer, polyoxyalkylene fatty acid monoisopropanol amide, sodium sulfate, sodium chloride and a mixture thereof.

Further, in addition to the cleansing component, it may further include an organic powder selected from polysaccharides, dimethyl polysiloxane, polymethyl methacrylate and nylon, or an inorganic powder selected from silica, kaolin, zinc oxide, titanium oxide, zeolite and calcium carbonate, or a mixture thereof, which contributes to preventing clumping in the process of dissolving the bulk cleaning component and enhancing the cleaning effect.

Further, in addition to the cleaning component, it may further include at least one thickener selected from the group consisting of polyacrylic acid or a derivative thereof, polyvinyl alcohol, methyl cellulose, hydroxymethyl cellulose, xanthan gum, alginic acid, carrageenan and a mixture thereof, which stabilizes the generated foam and prevents it from being washed off excessively quickly with water from the hair or skin.

In addition, as the conditioning component, hydrocarbon oils such as dimethicone, cyclomethicone, amination silicone, and isoparaffin, and ester oils such as isopropyl palmitate and isostearyl isostearate can be used together.

Further, it may further include commonly used preservatives, pH adjusters, fragrances, dyes, and the like, together with hinokitiol, menthol, salicylic acid, zinc pyrithione, and tocopherol acetate, which are known to have a hair-growth effect.

In the present invention, the structure of the skin layer is important to provide a beautiful form of solid personal care products and to provide packaging, portability and convenience of use. The function of the skin layer is to minimize the loss of fragrance and volatile conditioning components contained in the core layer during the drying process, while being easily hydrated with water. When commercialized, there is no scattering powder containing the surfactant that has come off from the core of the product. Further, not only it prevent the products from sticking to each other or deforming the shape of the product even in a high-humidity environment, but also when using the product, it can obtain ancillary effects such as improving the quality of the generated foam and allowing the foam to be maintained for a long time.

The water-soluble or water-dispersible polymer contained in the skin layer is easily dissolved in water, has a film-forming ability, and has excellent compatibility with the core layer. The water-soluble or water-dispersible polymer may be at least one selected from the group consisting of polyvinyl alcohol, alginic acid, polyacrylic acid, polyvinylacrylamide, polyvinylpyrrolidone, a cellulose derivative and a mixture thereof.

Further, the skin layer may further include a natural fibrous polymer selected from chitin, chitosan, cellulose and silk, or a synthetic fibrous polymer selected from acrylic, nylon, rayon and polyester, or a mixture thereof.

In order to form the skin layer, the above-listed water-soluble or water-dispersible polymer and the above-listed fibrous polymer may be mixed to form a film or paper, which may be configured as a single layer, and it is composed of a multi-layer structure laminated in a film state or coated on a fibrous polymer layer on a film or paper, so that while the bulk component of the core layer is drying, but the evaporation of moisture is facilitated, but it is possible to produce a functional solid personal care product composed of a functional skin layer that minimizes the loss of fragrance or functional components.

Specifically, the method of forming the skin layer may include a method of coating the surface of the bulk core layer, or a method of using a pre-prepared monolayer or multilayer film as the skin layer, but containing the bulk core layer between the skin layer films.

Further, the method of coating the surface of the bulk core layer can be roughly divided into two. First, the water-soluble or water-dispersed bulk (cleaning or conditioning component) is frozen to form a core layer. On the other hand, a water-soluble or water-dispersible polymer having film-forming ability is dissolved or dispersed in water or a water/solvent mixture, which is coated onto the surface of the frozen core layer by roll coating or spray coating method, then frozen together, then put in a freeze dryer again and cut as it is in a freeze-dried state to obtain a solid personal care product.

Further, in another method of coating the surface of the bulk core layer, a dry bulk core layer having a certain shape is formed by general drying or freeze-drying. Then, as described above, a water-soluble or water-dispersible polymer having a film-forming ability is dissolved or dispersed in water or a water/solvent mixture, which is coated and dried on the surface of the core layer on the dry bulk in a tablet coating method for the production of a dragee or a coated tablet, thereby capable of obtaining a solid personal care product.

According to the above two coating methods, the shape is relatively simple and the size is not large like a spherical shape, a tablet type, and a chocolate type, and thus is suitable for personal care products such as cleansing foam, shaving foam, makeup remover, and facial cleanser.

The skin layer may be configured such that a water-soluble paper or a water-soluble polymer film coated with a water-soluble polymer, or a water-soluble paper laminated with a water-soluble polymer film is laminated on both surfaces of the core layer.

That is, a pre-prepared single-layer or multi-layer film is used as the skin layer, wherein a bulk core layer is contained between skin layer films. The film for the skin layer is a skin structure composed of a water-soluble component containing a water-soluble or water-dispersible fiber, and functions so as to maintain the shape of the care product and facilitate packaging of the finally produced product.

The water-soluble polymer film may be at least one selected from the group consisting of polyvinyl alcohol, alginic acid, polyacrylic acid or a derivative thereof, and polyacrylamide, which dissolves or swells even in high humidity conditions so that it does not stick to each other, and it is possible to print for design.

The film/sheet-like skin layer that can be processed in this way is suitable for producing sandwich type products in which the upper and lower parts of the core layer are attached with skin layer films and then cut. Further, it is suitable for the production of sealed products that are cut after sealing the ends of the four edges using heat, ultrasonic waves, high frequency or induction heating with the bulk of the skin layer film being contained, wherein an active component having a low volatilization temperature can be retained. In addition, mass production is possible with the wrapping packaging method of packaging in the form of a sachet or pouch, which is packaged using a packaging machine that uses the existing flexible packaging film. Further, it is preferable for producing solid personal care cleaning products such as body cleanser, hair shampoo, hair conditioner, and hair essence having a relatively large capacity.

Further, the present invention provides a method for producing a solid personal care product, comprising the steps of: (I) freeze-drying a liquid bulk component containing a cleaning or conditioning component to form a core layer; and (II) coating the core layer with a solution containing water-soluble or water-dispersible polymers, followed by freeze-drying to form a coating layer.

Further, the present invention provides a method for producing a solid personal care product, comprising the steps of: (I) coating a liquid bulk component containing a cleaning or conditioning component onto a water-soluble paper coated with a water-soluble polymer, and then laminating the water-soluble paper coated with the water-soluble polymer thereon; and (II) drying the resulting laminate with a hot air dryer to form a skin layer in which a water-soluble paper coated with a water-soluble polymer is laminated on both surfaces of a core layer containing a cleaning or conditioning component.

Further, the present invention provides a method for producing a solid personal care product, comprising the steps of: (I) coating a bulk slurry containing a cleaning or conditioning component in a state of -5°C to -10°C onto a water-soluble paper or a water-soluble polymer film coated with a water-soluble polymer in a chamber maintained at -30°C to -50°C, and then laminating a water-soluble paper or a water-soluble polymer film coated with a water-soluble polymer thereon; and (II) drying the resulting laminate with a freeze dryer to form a skin layer in which a water-soluble paper or a water-soluble polymer film coated with a water-soluble polymer is laminated on both surfaces of a core layer containing a cleaning or conditioning component.

Further, the present invention provides a method for producing a solid personal care product, comprising the steps of: (I) coating a bulk slurry containing a cleaning or conditioning component in a state of -5°C to -10°C onto a composite film in which a water-soluble polymer film is laminated on a water-soluble paper in a chamber maintained at -30°C to -50°C; and (II) tightly adhering the film surface of the composite film so as to go to the inside and then freeze-drying to form a skin layer in which the composite film is laminated on both surfaces of a core layer containing a cleaning or conditioning component.

The solid personal care product according to the present invention can be produced in various shapes such as spherical shape, disc shape, polygonal shape, hexahedral shape, sandwich-shape or plate-shape.

Hereinafter, the present invention will be described in detail by way of examples and comparative examples.

### Example

First, a bulk component for forming the core layer as shown in Table 1 below was prepared.

**Table 1**

| Bulk component | Bulk A | Bulk B | Bulk C |
|---|---|---|---|
| Sodium lauryl sulfate (SLS) | - | 5.0 | 5.0 |
| Sodium laureth sulfate (SLES) | 10 | 20.0 | 20.0 |
| Cocobetaine | 5 | 3.0 | - |
| Coconut mono-ethanol amide | 8 | - | - |
| Isopropyl myristate | 5 | 1 | 3 |
| Starch | 20.0 | 10.0 | 11.0 |
| S sodium sulfate | 30.0 | 12 | 12 |
| Sodium chloride | - | 3.0 | 2.0 |
| Glycerine | 5.0 | 3.0 | 2.5 |
| Citric acid | 0.1 | 0.2 | 0. |
| Polyethylene glycol (PEG-8) | 5.0 | 8.0 | 10.0 |
| Lavender essential oil | - | - | 3 |
| Water | 11.9 | 34.8 | 31.3 |
| Total (wt %) | 100.0 | 100.0 | 100.0 |

### Example 1

Bulk B of Table 1 was filled in a silicone rubber mold having a ball shape (spherical shape) with a diameter of 15 mm, frozen, and then the temperature of the ball was maintained at -35°C. A 3 wt% polyvinyl alcohol aqueous solution (using a mixture of 85 wt% purified water/15 wt% ethanol as a solvent) was spray-coated to a thickness of 0.3 mm onto the surface of the ball, frozen, and then freeze-dried to produce a cleaning agent having a ball shape (spherical shape).

### Example 2

Bulk B of Table 1 was filled in a silicone rubber mold having a ball shape (spherical shape) with a diameter of 15 mm, frozen, and then the temperature of the ball was maintained at -35°C. An aqueous solution of 3 wt% polyvinyl alcohol and 0.5 wt% cellulose nanofiber (using a mixture of 85 wt% purified water/15 wt% ethanol as a solvent) was spray-coated to a thickness of 0.3 mm onto the surface of the ball, frozen, and then freeze-dried to produce a cleaning agent having a ball shape (spherical shape).

### Example 3

A 4 wt% polyvinyl alcohol aqueous solution was coated in an amount of 5 g/m² onto 30 g/m² of water-soluble paper and dried to obtain paper/coated paper. The bulk A of Table 1 was coated to a thickness of 1.5 mm onto the paper/coated paper, and then the paper/coated paper was covered and laminated thereon, dried in a hot air dryer at 80°C to obtain a sandwich-type cleaning agent having a moisture content of 5 wt%, which was cut into a size of 5 cm x 4 cm to produce a cleaning agent having a sandwich structure.

### Example 4

The paper/coated paper according to Example 3 was placed on a chamber maintained at -30°C, and then the bulk C slurry of Table 1 was coated thereon to a thickness of 1.5 mm at -5°C, and then the paper/coated paper was placed again, tightly adhered, frozen, and freeze-dried to obtain a sandwich-type cleaning agent, which was cut into a size of 4 cm x 5 cm to produce a cleaning agent having a sandwich structure.

### Example 5

Water-soluble polyvinyl alcohol film (30 g/m²) was passed through a heating roll on 30 g/m² water-soluble paper, and laminated to obtain a paper/film laminate. The paper/film laminate was placed on a chamber maintained at -30°C, and the bulk C slurry of Table 1 was coated thereon to a thickness of 1.5 mm at -5°C, and the paper/film laminate was tightly adhered with the film surface as to as go to the inward, frozen, and freeze-dried to produce a cleaning agent having a sandwich structure. The outside of the portion where the bulk C of the frozen cleaning agent having a sandwich structure was contained was sealed with a line having a thickness of 1.5 mm, which was freeze-dried and cut into an outer size of 6 cm x 7 cm to produce a cleaning agent of which four surfaces were sealed.

### Example 6

2 wt% polyvinyl alcohol and 1 wt% cellulose nanofiber aqueous solution was coated at a coating amount of 8 g/m² on 30 g/m² of polyvinyl alcohol film and dried with hot air to obtain a coating/film. The coating/film was placed on a chamber maintained at -30°C, and the bulk C slurry of Table 1 was coated thereon to a thickness of 1.5 mm at -5°C, and then tightly adhered with the film surface of the coating/film so as to go to the inside, frozen, and freeze-dried to produce a cleaning agent having a sandwich structure. The outside of the portion where the bulk C of the frozen cleaning agent having a sandwich structure was contained was sealed with a line having a thickness of 1.5 mm, which was freeze-dried and cut into an outer size of 6 cm x 7 cm to produce a cleaning agent of which four surfaces were sealed.

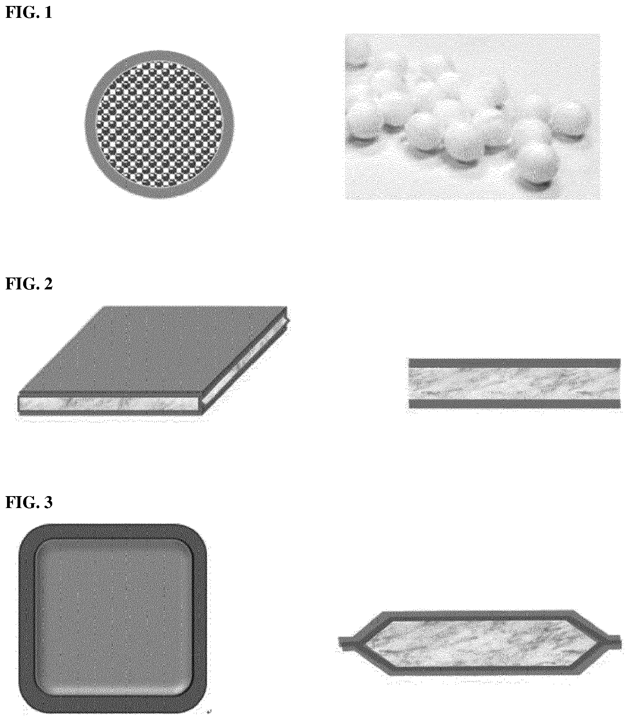

FIGS. 1 to 3 show a schematic diagram of the solid cleaning agent produced in Examples 1 to 6 above.

### Comparative Example 1

The bulk B of Table 1 was filled in a silicone rubber mold having a ball shape (spherical shape) with a diameter of 15 mm, and frozen. Freeze-drying was started at -30°C, and the freeze-drying was completed at -50°C, thereby producing a ball-shaped (spherical) cleaning agent having a moisture content of 7% by weight or less.

### Comparative Example 2

The bulk C of Table 1 was filled in a 4 cm × 5 cm silicon mold with a depth of 1.5 mm and frozen. Then, freeze-drying was started at -30°C, and the freeze-drying was completed at -50°C, thereby producing a flat square cleaning agent having a moisture content of 7% by weight or less.

Table 2 below shows the evaluation results of the solubility, foaming power, clumping, storability, and fragrance-retaining property of the solid cleaning agents produced from Examples 1 to 6 and Comparative Examples 1 and 2.

**Table 2**

| Sample | ①Solubility | ②Foaming power | ③Clumping | ④Storability | ⑤Fragrance-retaining property |
|---|---|---|---|---|---|
| Example 1 | Excellent | Good | Good | Ordinary | - |
| Example 2 | Good | Good | Good | Good | - |
| Example 3 | Bad | Normal | Bad | Good | - |
| Example 4 | Good | Normal | Good | Good | Normal |
| Example 5 | Good | Good | Ordinary | Good | Good |
| Example 6 | Good | Good | Good | Ordinary | Good |
| Comparative Example 1 | Excellent | Normal | Normal | Bad | - |
| Comparative Example 2 | Excellent | Normal | Good | Bad | Bad |

Note) ① Solubility: Time taken for 50 wt% or more of morphology to collapse when immersed in purified water at 20°C (within 3 seconds: excellent, within 5 seconds: good, within 10 seconds: normal, 10 seconds or more: bad ).

② Foaming power: Rub the cleaning agent by hand and sensually measure whether it foams well or maintains the foam well (poor in foamability: bad; having foamability and low in sustainability: normal; good in foamability and good in sustainability: good).

③ Clumping: When rubbing the cleaning agent by hand, the degree of lump formation is sensually measured (remains in a solid state until the end, lasts for 20 seconds or more: bad; a lump exist, but disappears at the end, and lasts for 10 to 20 seconds: normal; the lump disappears within 10 seconds: good).

④ Storability: After leaving the product at 40°C and 70% relative humidity for 24 hours, observe the sticking of the products and the deformation of the shape (no change in shape or size: good, sticking to each other, but when removed, it separates again: normal, it changes shape or sticks to each other and cannot be separated: bad).

⑤ Fragrance-retaining property: When used, the degree of scent emission is sensually measured (the scent is strong and rich: good; bulk-based odor does not exist but scent is faint: normal; bulk based odor is strong and scent is faint: bad).

Further, as a particular matter other than those described in Table 2 above, the solid cleaning agent produced from Examples 1 to 6 of the present invention are relatively excellent in foaming power and appearance, and have excellent commerciality. On the other hand, the solid cleaning agents produced from Comparative Examples 1 and 2 were evaluated to have a fragile surface, difficult to maintain their morphology, and lacked commerciality.

Therefore, the solid personal care products produced by the present invention solves the disadvantage that existing conventional solid cleansing agents or conditioning agents in powder, soap, or other flake form have of the powder scattering, as well as the problem of the cleansing power, conditioning efficacy, and fragrance-preserving property of such agents degrading. Moreover, the contents of the cleansing or conditioning component does not rub off onto the hands of a user even when the user holds the contents in the user's hands, and the shape of the product according to the present invention does not change even in places of high humidity. In addition, this solid personal care product is easy to use, store, and package, while retaining the same functions as those of conventional liquid cleansing and conditioning personal care products.

## Claims

1. A solid personal care product comprising: a core layer containing a cleansing or conditioning component; and a skin layer containing a water-soluble or water-dispersible polymer.

2. The solid personal care product according to claim 1, wherein the cleansing component is at least one selected from the group consisting of sodium alkyl sulfate having 12 to 14 carbon atoms, alkyl monoethanol amide having 12 to 14 carbon atoms, N-acyl glutamate, amphoteric surfactant, cationic water-soluble polymer, polyoxyalkylene fatty acid monoisopropanol amide, sodium sulfate, sodium chloride and a mixture thereof.

3. The solid personal care product according to claim 2, wherein the cleansing component further comprises an organic powder selected from polysaccharides, dimethyl polysiloxane, polymethyl methacrylate and nylon, or an inorganic powder selected from silica, kaolin, zinc oxide, titanium oxide, zeolite and calcium carbonate, or a mixture thereof.

4. The solid personal care product according to claim 2, wherein the cleansing component further comprises at least one thickener selected from the group consisting of polyacrylic acid or a derivative thereof, polyvinyl alcohol, methyl cellulose, hydroxymethyl cellulose, xanthan gum, alginic acid, carrageenan and a mixture thereof.

5. The solid personal care product according to claim 1, wherein the conditioning component is at least one selected from the group consisting of dimethicone, cyclomethicone, aminated silicone, isoparaffin, isopropyl palmitate, isostearyl isostearate and a mixture thereof.

6. The solid personal care product according to claim 1, wherein the water-soluble or water-dispersible polymer is at least one selected from the group consisting of polyvinyl alcohol, alginic acid, polyacrylic acid, polyvinylacrylamide, polyvinylpyrrolidone, a cellulose derivative and a mixture thereof.

7. The solid personal care product according to claim 1, wherein the skin layer further comprises a natural fibrous polymer selected from chitin, chitosan, cellulose and silk, or a synthetic fibrous polymer selected from acrylic, nylon, rayon and polyester, or a mixture thereof.

8. The solid personal care product according to claim 1, wherein the skin layer is a coating layer formed on a surface of the core layer.

9. The solid personal care product according to claim 1, wherein the skin layer is configured such that a water-soluble paper or a water-soluble polymer film coated with a water-soluble polymer, or a water-soluble paper laminated with a water-soluble polymer film is laminated on both surfaces of the core layer.

10. The solid personal care product according to claim 9, wherein the water-soluble polymer film is at least one selected from the group consisting of polyvinyl alcohol, alginic acid, polyacrylic acid or a derivative thereof and polyacrylamide.

11. The solid personal care product according to any one of claims 1 to 10, wherein the solid personal care product is spherical, disc-shaped, polygonal, hexahedral, sandwich-shaped or plate-shaped.

12. A method for producing a solid personal care product, comprising the steps of:
(I) freeze-drying a liquid bulk component containing a cleaning or conditioning component to form a core layer; and
(II) coating a solution containing a water-soluble or water-dispersible polymer onto the core layer, followed by freeze-drying to form a coating layer.

13. A method for producing a solid personal care product, comprising the steps of:
(I) coating a liquid bulk component containing a cleaning or conditioning component onto a water-soluble paper coated with a water-soluble polymer, and then laminating the water-soluble paper coated with the water-soluble polymer thereon; and
(II) drying the result with a hot air dryer to form a skin layer in which a water-soluble paper coated with a water-soluble polymer is laminated on both surfaces of a core layer containing a cleaning or conditioning component.

14. A method for producing a solid personal care product, comprising the steps of:
(I) coating a bulk slurry containing a cleaning or conditioning component in a state of -5°C to -10°C onto a water-soluble paper or a water-soluble polymer film coated with a water-soluble polymer in a chamber maintained at -30°C to -50°C, and then laminating a water-soluble paper or a water-soluble polymer film coated with a water-soluble polymer thereon; and
(II) drying the result with a freeze dryer to form a skin layer in which a water-soluble paper or a water-soluble polymer film coated with a water-soluble polymer is laminated on both surfaces of a core layer containing a cleaning or conditioning component.

15. A method for producing a solid personal care product, comprising the steps of:
(I) coating a bulk slurry containing a cleaning or conditioning component in a state of -5°C to -10°C onto a composite film in which a water-soluble polymer film is laminated on a water-soluble paper in a chamber maintained at -30°C to -50°C; and
(II) tightly adhering the film surface of the composite film so as to go to the inside and then freeze-drying to form a skin layer in which the composite film is laminated on both surfaces of a core layer containing a cleaning or conditioning component.

16. The method for producing a solid personal care product according to claim 14 to 15, wherein
the ends of the four edges are sealed using heat, ultrasonic waves, high frequency or induction heating with the bulk of the skin layer film embedded therein, then cut and sealed.
